# EUROPEAN PATENT APPLICATION

(11) **EP 3 521 305 A1**
(43) Date of publication of application: **07.08.2019**
(21) Application number: 18000103.4
(22) Date of filing: 06.02.2018
(51) Int. Cl.: C07K 14/34, C12P 21/00, C12P 13/04

(54) **CORYNEBACTERIUM SPP. STRAINS HAVING INCREASED RATES OF GROWTH AND/OR SUGAR UPTAKE**

(71) Applicant: Universität Stuttgart, 70174 Stuttgart (DE)
(72) Inventor: The designation of the inventor has not yet been filed
(74) Representative: Müller-Boré & Partner Patentanwälte PartG mbB

(57) **Abstract**

The present invention relates to recombinant *Corynebacterium* spp. host cells comprising, in relation to wild-type cells, at least one mutation selected from the group consisting of mutations that (i) reduce the activity of a protein, selected from the group consisting of RamA, SigH, RshA, SigM, SufR, OxyR, SigE, SigA, and SigD, or (ii) increase the activity of a protein, selected from the group consisting of RipA and DtxR. Said recombinant host cells are characterized by increased growth and/or sugar uptake rates, even showing increased sugar uptake rates without a proportional increase in growth rates, that lead to increased productivity when using said cells for the production of biosynthetic products. The present invention further relates to respective methods for the biosynthetic production of a product of interest using said host cells.

## Description

The present invention relates to recombinant *Corynebacterium* spp. host cells comprising, in relation to wild-type cells, at least one mutation selected from the group consisting of mutations that (i) reduce the activity of a protein, selected from the group consisting of RamA, SigH, RshA, SigM, SufR, OxyR, SigE, SigA, and SigD, or (ii) increase the activity of a protein, selected from the group consisting of RipA and DtxR. Said recombinant host cells are characterized by increased growth and/or sugar uptake rates, even showing increased sugar uptake rates without a proportional increase in growth rates, that lead to increased productivity when using said cells for the production of biosynthetic products. The present invention further relates to respective methods for the biosynthetic production of a product of interest using said host cells.

*Corynebacterium glutamicum* (*C. glutamicum*) is a bacterium heavily used in industrial biotechnology, e.g. in the production of amino acids. The amount of amino acids produced in *C. glutamicum* surpasses two million tons per annum. This encompasses products such as L-glutamic acid, L-lysine and other L-amino acids finding a range of applications in biotechnology or the food and feed industry.

The production of low-molecular products in bacteria can be closely coupled, partially coupled or decoupled with/from the growth of the production bacteria. As a host for the production of closely or partially growth-coupled products, *C. glutamicum* exhibits the intrinsic disadvantage that it's maximum growth rate of < 0.6 h⁻¹ in complex media and < 0.3 h⁻¹ in synthetic media is significantly lower than that of e.g. *Escherichia coli.*

These relatively low growth rates and resulting low cell specific productivity leads to a lower volumetric productivity which so far can be countered only by using higher biomass concentrations or be using entirely different production systems. Thus, due to the lower productivity, the commercial potential cannot be fully realized. Further, increased biomass concentrations reduces the factual product yield. Furthermore, when forced to use different production systems, the advantages of *C. glutamicum* as industrial production strain, such as its superior robustness, cannot be utilized.

So far, the genes encoding the above regulatory proteins have not yet been analyzed in a target-oriented manner with respect to increasing the growth rate and sugar uptake rate of *C. glutamicum.* In particular, there are no known mutations or targeted modifications in said bacterium that would specifically lead to such increased rates.

Raising the intrinsically low growth rate and sugar uptake rate of *C. glutamicum* would open new fields of application with higher production rates for this bacterium. This would include the growth-coupled production of higher-molecular products such as proteins.

Therefore, the technical problem underlying the present invention is the provision of recombinant *Corynebacterium* spp. host cells displaying significantly increased growth and sugar uptake rates, as well as production processes using the same.

The solution to the above technical problem is achieved by the embodiments characterized in the claims.

In particular, in a first aspect, the present invention relates to a recombinant *Corynebacterium* sp. host cell, wherein said cell comprises at least one mutation in relation to a wild-type cell, wherein said mutation is selected from the group consisting of mutations that
(i) reduce the activity of a protein, selected from the group consisting of RamA, SigH, RshA, SigM, SufR, OxyR, SigE, SigA, and SigD; or
(ii) increase the activity of a protein, selected from the group consisting of RipA and DtxR.

As used herein, the term "recombinant cell" refers to a cell whose genome has been artificially altered as compared to a wild-type cell.

*Corynebacterium* sp. that are encompassed in the present invention are not particularly limited and respective bacteria are known in the art. In preferred embodiments, the recombinant host cell is a *C. glutamicum* host cell, preferably derived from *C. glutamicum* strain *C. glutamicum* ATCC 13032.

The term "mutation" as used herein relates to any permanent alteration of the nucleotide sequence of the genome of the host cell. Further, the term "mutation in relation to a wild-type cell" as used herein relates to the fact that said mutation is not present in a wild-type cell, but is present in the host cells of the present invention.

In embodiments wherein the recombinant host cell of the present invention is a *C. glutamicum* host cell, *C. glutamicum* wild-type is *C. glutamicum* strain *C. glutamicum* ATCC 13032.

The mutation present in the host cells of the present invention is selected from the group consisting of mutations that
(i) reduce the activity of a protein, selected from the group consisting of RamA, SigH, RshA, SigM, SufR, OxyR, SigE, SigA, and SigD, preferably from the group consisting of RamA, SigH, RshA, SigM, SufR, OxyR, and SigE; or
(ii) increase the activity of a protein, selected from the group consisting of RipA and DtxR.

More preferably, said mutation is selected from the group consisting of mutations that
(i) reduce the activity of the transcriptional regulator RamA (Regulator of acetate metabolism A); or
(ii) increase the activity of the transcriptional regulator DtxR /Diphteria toxin repressor).

In a particularly preferred embodiment, said mutation is the amino acid substitution S101C in RamA (RamA[S101C]); or the amino acid substitution R103H in DtxR (DtxR[R013H]).

Mutations that reduce or increase the activity of a protein are not particularly limited and are known in the art. They include amino acid substitutions, additions and deletions, as well as deletion of the gene encoding the respective protein. The term "deletion" as used herein relates to the removal of any number of nucleotides that leads to a complete abolishment of the expression of functionally active protein. By way of example, deletion of a gene may encompass removal of the entire gene or the entire coding sequence, or removal of only few or a single nucleotide(s) leading to a complete abolishment of expression or a total loss of protein activity.

Methods for gene deletion in *Corynebacterium* spp. are not particularly limited and are known in the art. Further, methods for introducing amino acid substitutions, additions or deletions in a particular protein are not particularly limited and are known in the art. They include any methods of altering the respective coding sequence so that the modified protein instead of the wild-type protein is encoded.

RamA and DtxR are transcriptional regulators found in a wide range of bacteria, including *Corynebacterium* spp. and in particular *C*. *glutamicum,* and are encoded by the *ramA* and *dtxR* gene, respectively. Sequence information for *C. glutamicum* can be found for RamA under UniProtKB/Swiss-Prot Accession Number Q8NML3.1 and for DtxR under UniProtKB/Swiss-Prot Accession Number Q8NP95. Whereas RamA has so far only been associated with the regulation of the central metabolism with respect to growth on acetate, DtxR represents the master regulator of iron homeostasis in *C. glutamicum.*

SigH, RshA, SigM, SufR, OxyR, SigE, SigA, SigD, RipA and DtxR are encoded by the genes *sigH, rshA, sigM, sufR, oxyR, sigE, sigA, sigD, ripA* and *dtxR,* respectively. Respective sequence information for C. *glutamicum* can be found under GenBank accession numbers CAD32033.1 (*sigH*)*,* ASW13468.1 (*rshA*), CAD32030.1 (*sigM*)*,* ASW14175.1 (*sufR*), CAD32032.1 (*oxyR*), CAD32043.1 (*sigE*), CAA89984.1 (*sigA*), BAU95001.1 (*sigD*), and L35906.1 (*dtxR*). Sequence information for *C. glutamicum ripA* can be found under UniProtKB/Swiss-Prot Accession Number Q8NRR3.

In a particular embodiment, the host cell of the present invention is a recombinant *C. glutamicum* host cell, wherein said cell comprises, in relation to a wild-type cell, *i.e.,* in relation to *C. glutamicum* strain *C. glutamicum* ATCC 13032, (i) the amino acid substitution S101C in RamA (RamA[S101C]); or (ii) the amino acid substitution R103H in DtxR (DtxR[R013H]). Respective cells comprising RamA[S101C] are sometimes designated herein as *C. glutamicum* strain *C. glutamicum* FastA.

In a second aspect, the present invention relates to a method for the biosynthetic production of a product of interest (POI), wherein said POI is produced in a recombinant host cell according to the present invention.

The term "biosynthetic production" as used herein relates to the fact that the POI is produced via endogenous biosynthesis in the host cells of the present invention or by help of said host cells.

The POI does not underlie any specific restrictions, provided it can be biosynthetically produced in the host cells of the present inventions. In particular embodiments, the POI is a protein and said protein is expressed in the host cells of the present invention, wherein preferably the protein is a heterologous protein that is expressed in the host cells of the present invention in a growth-coupled manner. In other particular embodiments, the POI is an organic molecule and said organic molecule is produced in the host cells of the present invention as a metabolite. In preferred embodiments, the organic molecule is selected from the group consisting of amino acids, preferably L-amino acids, including e.g. L-glutamic acid, L-lysine, L-ornithine, L-proline, L-arginine, and L-citrulline, as well as derivatives thereof. In another preferred embodiment, the organic molecule is gamma-butyric acid (GABA).

As used herein the term "produced in the host cells as a metabolite" relates to the fact that the POI is the result of a particular metabolic pathway of the host cells of the present invention. This metabolic pathway may be an endogenous pathway that is already present in wild-type cells, or an engineered pathway that is implemented or modified transgenically. In preferred embodiments, the starting material for the production of said metabolite is a sugar, preferably a sugar selected from the group consisting of glucose, fructose, mannose, xylose, arabinose, and sucrose.

Preferably, the methods of the present invention as defined above comprise the steps of:
(a) providing a host cell of the present invention, wherein said host cell is capable of producing the POI,
(b) culturing said host cell under conditions allowing production of the POI.

Means of rendering a host cell capable of producing one or more particular POI do not underlie any specific restrictions and are known in the art. Further, respective culture techniques and conditions are known in the art. By way of example, such techniques include batch or continuous flow processes in bioreactors, fed-batch processes with our without cell retention, immobilized or submerged cultivated cells, and *Corynebacterium* sp. biofilms, optionally in an industrial scale.

In a third aspect, the present invention relates to the use of a recombinant host cell of the present invention for the biosynthetic production of a product of interest (POI).

In this aspect, all relevant definitions and limitations defined above for the host cells and the methods of the present invention apply in an analogous manner. The term "comprise(s)/comprising" as used herein is expressly intended to encompass the terms "consist(s)/consisting essentially of" and "consist(s)/consisting of".

The present invention provides *Corynebacterium* spp. host cells having increased growth and sugar uptake rates, resulting in higher volumetric productivity and higher cell-specific production rates. As demonstrated for the exemplary *C. glutamicum* strain *C. glutamicum* FastA, this can be achieved by targeted modification of RamA, in particular by introducing the single amino acid substitution S101C in RamA (RamA[S101C]). *C. glutamicum* FastA reaches growth rates of > 0.7 h⁻¹ in complex media and > 0.4 h⁻¹ in synthetic media, thus providing a growth rate increase of 15 to 30% as compared to the wild-type. Furthermore, a disproportionate increase of the biomass specific glucose uptake rate of 36% is observed which corresponds to a 25 % rise of the growth rate. The increased growth rate advantageously not only shortens the phase of accumulating biomass in production reactors, but also provides a respective increase in cell-specific productivity in growth-coupled production processes. The disproportionate raise of the glucose uptake rate provides improved availability of precursors even under moderate growth conditions which enables high substrate-to-product conversions via the precursor pyruvate. Since the mutations employed in the present invention are independent of the POI to be produced, the present invention provides a general interest production platform for all *Corynebacterium* spp.-based production processes.

The figures show:

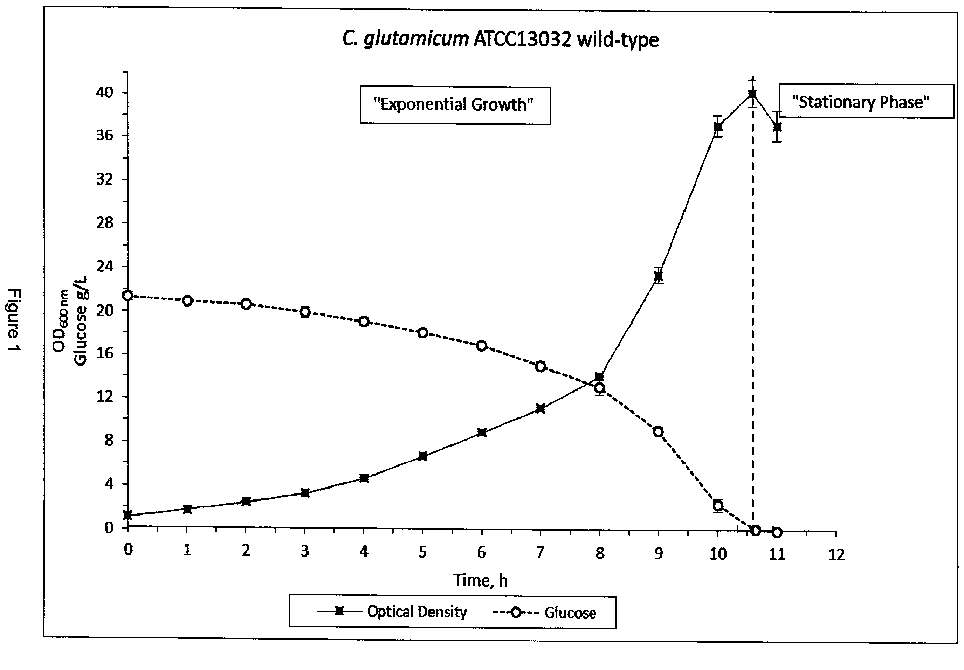

Figure 1: Batch cultivation of *Corynebacterium glutamicum* ATCC 13032 wild-type strain in a minimal medium (CGXII) supplemented with 20 g/L glucose as sole C-source. The strain grew exponentially until glucose was totally consumed by the strain at 10.5 h. Data points and error bars derive from three parallel fermentations n=3.

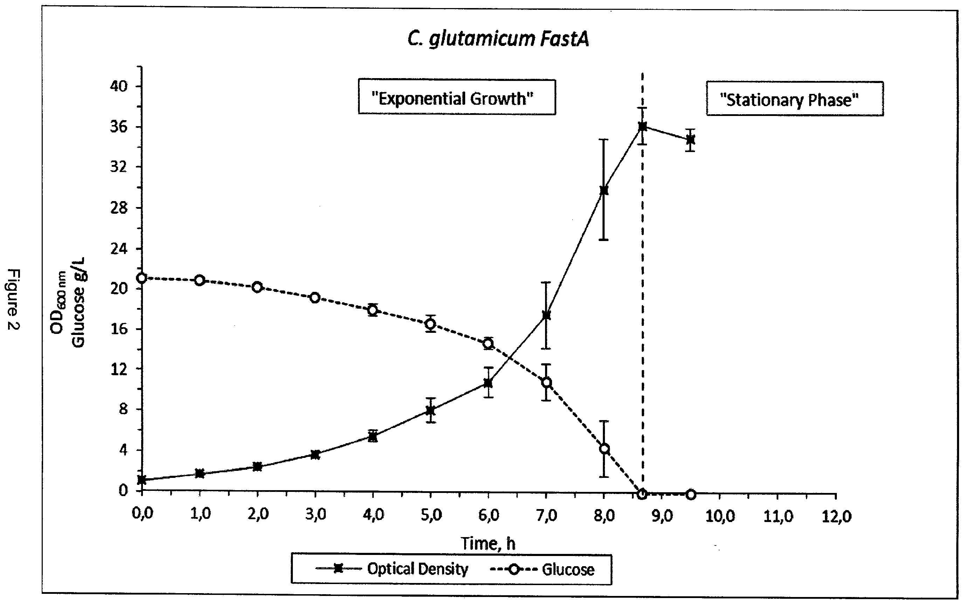

Figure 2: Batch cultivation of *Corynebacterium glutamicum* FastA strain in a minimal medium (CGXII) supplemented with 20 g/L glucose as sole C-source. The strain grew exponentially until glucose was totally consumed by the strain at 8.5 h. Data points and error bars derive from three parallel fermentations n=3.

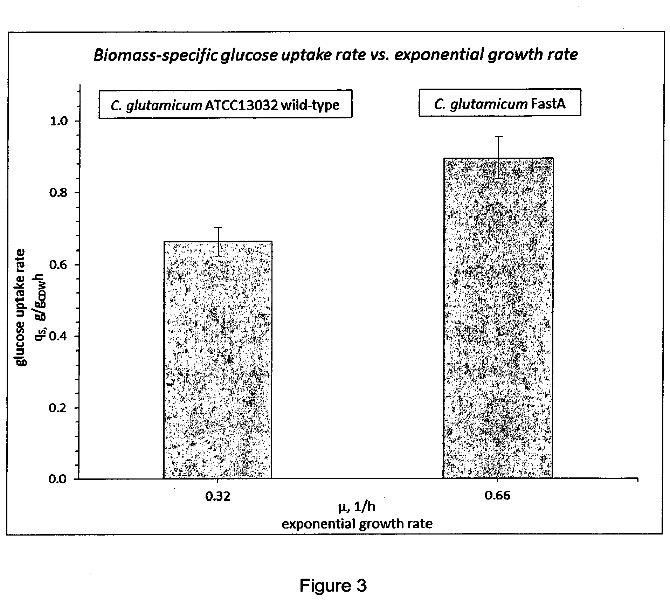

Figure 3: Biomass-specific glucose uptake rates (q_{S}) versus exponential growth rates (µ) of *Corynebacterium glutamicum* strains ATCC 13032 wild-type and FastA. The mutated strain *C. glutamicum* FastA exhibits an increased µ of 25% and an increased qs of 36%.

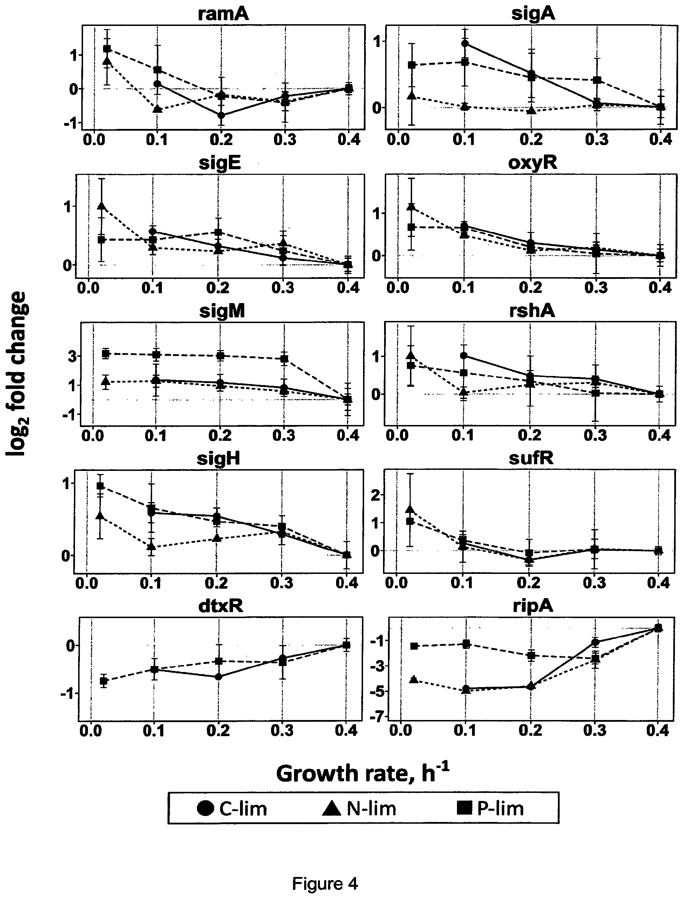

Figure 4: Expression profiles of chosen regulator genes over the growth rate in *C. glutamicum.* Regulators ramA, sigA, sigE, oxyR, sigM, rshA, sigH, sufR exhibit a decrease in expression levels with increasing growth rate. The regulators dtxR, ripA show an increase in expression level with increasing growth rate.

The present invention will be further illustrated by the following examples without being limited thereto.

### Examples

### Material and Methods:

### Re-Engineering of RamA(S101C).

To re-engineer the genomic *ramA* mutation (TCC->TGC) in *C. glutamicum* ATCC 13032 encoding RamA(S101C), the pK19mobsacB vector known in the art was applied. The 500 bp sequences flanking the point mutation were amplified with the primer pairs fw_ramA-flank1 (SEQ ID NO: 1) + rev_ramA-flank1 (SEQ ID NO: 2) (upstream region) and fw_ramA-flank2 (SEQ ID NO: 4) + rev_ramA-flank2 (SEQ ID NO: 4) (downstream region), respectively. The C->G mutation itself was encoded in the primer rev_ramA-flank1 and fw_ramA-flank2, respectively (cf. below, marked in bold).

Both 500 bp amplification products were inserted simultaneously in Hindlll and BamHI linearized pK19mobsacB by isothermal assembling as known in the art, yielding pK19mobsacB-*ramA*(S101C).

Plasmids were amplified in *E. coli* DH5α cells after electroporation and purified using a commercially available kit (*E.Z.N.A. Plasmid Mini kit I,* Omega Bio-Tek Inc., Norcross, Georgia, USA).

Correct assembling of vector backbone and insert was double-checked by sequencing of the entire insert with primers fw_pK19-seq (SEQ ID NO: 5) and rev_pK19-seq (SEQ ID NO: 6) (GATC light run service).

The point mutation was eventually introduced in the genomic background of C. *glutamicum* ATCC 13032 by a cassette exchange using pK19mobsacB-*ramA*(S101C) as known in the art. Electrocompetent cells of *C. glutamicum* ATCC 13032 were prepared as known in the art. Briefly, cultures were harvested at an OD₆₀₀ of 1.75 and washed three times with 10 % (v/v) glycerol. Transformation of competent *C. glutamicum* cells with 5 µL of purified plasmid by electroporation (Eppendorf Eporator, 2500 V, 2 mm gap width, achieving time constants of 4.3 to 4.7 ms) was carried out following an optimized protocol known in the art.

Strains carrying the correct *ramA* mutation were identified by sequencing the PCR product of the mutated region amplified with primers fw_ramA-flank1 and rev_ramA-flank2. For sequencing the primer pair fw_ramA-seq and rev_ramA-seq was used.

**Table 1: Primers**

| **Primer** | **Sequence (5'->3')** | **SEQ ID NO.** |
|---|---|---|
| Fw_ramA-flank1 | AACAGCTATGACCATGATTACGCCAAGCTTGGGGGTTAACTACCTCTTCGG | 1 |
| Rev_ramA-flank1 | CGACAATGCAATGAAGGCCC | 2 |
| Fw_ramA-flank2 | GGGCCTTCATTGCATTGTCG | 3 |
| Rev_ramA-flank2 | AGTGAATTCGAGCTCGGTACCCGGGGATCCTTAAGGCAGTGCGCCGATC | 4 |
| Fw_pK19-seq | CAGGCTTTACACTTTATGC | 5 |
| Rev_pK19-seq | CCCATCTCTTCAGCAGC | 6 |
| Fw_ramA-seq | GTTGCAGGACAATCGCC | 7 |
| Rev_ramA-seq | CCCATCTCTTCAGCAGC | 8 |

*Media for Batch cultivations with C. glutamicum wild-type and FastA.*

### Media and Solutions - Complex medium for agar plates (2 × TY-medium)

| | |
|---|---|
| Tryptone | 16.00 g/L |
| Yeast Extract | 10.00 g/L |
| NaCl | 5.00 g/L |
| Agar | 18.00 g/L |

All components described above were dissolved in ionized water and sterilized for 20 min at 121 °C. The still warm solution was poured into sterile into petri dishes and was allowed to solidify.

### Media and solutions - Complex medium for reaction tubes (2 × TY-medium)

| | |
|---|---|
| Tryptone | 16.00 g/L |
| Yeast Extract | 10.00 g/L |
| NaCl | 5.00 g/L |

All components of the 2 × TY complex medium described above were dissolved in deionized water, aliquoted to 5 mL in glass reaction tubes and sterilized for 20 min at 121 °C.

### Media and solutions - Sterilization buffer

| | |
|---|---|
| K₂HPO₄ | 174.20 g/L |
| KH₂PO₄ | 136.09 g/L |

All components described above were dissolved in deionized water.

### Media and Solutions - Base

| | |
|---|---|
| NH₄OH | 25 % [V/V] |

### Media and solutions - Preparation of minimal medium MM1 (CGXII-medium)

Solution A: Salts

| | |
|---|---|
| (NH₄)₂SO₄ | 1.64 g/L |
| Urea | 0.82 g/L |
| MOPS | 3.44 g/L |
| K₂HPO₄ | 0.16 g/L |
| KH₂PO₄ | 0.16 g/L |

pH was adjusted to pH 7.0 with 5M KOH
Solution B: (1000 × Ca²⁺)

| | |
|---|---|
| CaCl₂ · H₂O | 10.00 g/L |

Solution C: (1000 × Mg²⁺)

| | |
|---|---|
| MgSO₄ · 7 H₂O | 250.00 g/L |

Solution D: (1000 × Trace Elements Solution = TES)

| | |
|---|---|
| Fe(II)SO₄ · 7 H₂O | 16.40 g/L |
| MnSO₄ · H₂O | 10.00 g/L |
| CuSO₄ · 5 H₂O | 0.20 g/L |
| ZnSO₄ · 7 H₂O | 1.00 g/L |
| NiCl₂ · 6 H₂O | 0.02 g/L |

Solution E: (1000 × Vitamin)

| | |
|---|---|
| Biotin | 0.20 g/L |

Solution F: (50 % w/v glucose)

| | |
|---|---|
| α-D(+)-Glucose · H₂O | 550.00 g/L |

To prepare the pre-culture minimal medium MM1, solutions A, B, C, and F described above were prepared separately and also separately sterilized at 121 °C for 20 min. Solutions D and E were separately prepared and sterile filtrated at 0.2 µm pore size. For 150 mL of ready to use pre-culture medium MM1, 137.4 mL salts, 0.15 mL 1000 × Ca²⁺ stock solution, 0.15 mL 1000 × Mg²⁺ stock solution, 0.15 mL 1000 × TES, 0.15 mL 1000 × Vitamin stock solution, and 12 mL 50 % w/v glucose were mixed.

### Media and solutions - Preparation of minimal medium MM2 (CGXII-medium)

Solution A: Salts

| | |
|---|---|
| (NH₄)₂SO₄ | 8.26 g/L |
| K₂HPO₄ | 0.83 g/L |
| KH₂PO₄ | 0.83 g/L |

Solution B: (1000 × Ca²⁺)

| | |
|---|---|
| CaCl₂ · H₂O | 10.00 g/L |

Solution C: (1000 × Mg²⁺)

| | |
|---|---|
| MgSO₄ · 7 H₂O | 250.00 g/L |

Solution D: (1000 × Trace Elements Solution = TES)

| | |
|---|---|
| Fe(II)SO₄ · 7 H₂O | 16.40 g/L |
| MnSO₄ · H₂O | 10.00 g/L |
| CuSO₄ · 5 H₂O | 0.20 g/L |
| ZnSO₄ · 7 H₂O | 1.00 g/L |
| NiCl₂ · 6 H₂O | 0.02 g/L |

Solution E: (1000 × Vitamin)

| | |
|---|---|
| Biotin | 0.20 g/L |

Solution F: (50 % w/v glucose)

| | |
|---|---|
| α-D(+)-Glucose · H₂O | 550.00 g/L |

To prepare the minimal medium MM2, solutions A, B, C, and F described above were prepared separately and also separately sterilized at 120 °C for 20 min. Solutions D and E were separately prepared and sterile filtrated at 0.2 µm pore size. For 714 mL of ready to use minimal medium MM2, 696 mL salts, 0.75 mL 1000 × Ca²⁺ stock solution, 0.75 mL 1000 × Mg²⁺ stock solution, 0.75 mL 1000 × TES, 0.75 mL 1000 × Vitamin stock solution, and 30 mL 50 % w/v glucose were mixed.

### Media and solutions - Preparation of minimal medium MM3 (CGXII-medium N-lim)

Solution A: Salts

| | |
|---|---|
| (NH₄)₂SO₄ | 2.19 g/L |
| MOPS | 45.90 g/L |
| K₂HPO₄ | 1.09 g/L |
| KH₂PO₄ | 1.09 g/L |

pH was adjusted to pH 7.5 with 5M KOH
Solution B: (1000 × Ca²⁺)

| | |
|---|---|
| CaCl₂ · H₂O | 10.00 g/L |

Solution C: (1000 × Mg²⁺)

| | |
|---|---|
| MgSO₄ · 7 H₂O | 250.00 g/L |

Solution D: (1000 × Trace Elements Solution = TES)

| | |
|---|---|
| Fe(II)SO₄ · 7 H₂O | 16.40 g/L |
| MnSO₄ · H₂O | 10.00 g/L |
| CuSO₄ · 5 H₂O | 0.20 g/L |
| ZnSO₄ · 7 H₂O | 1.00 g/L |
| NiCl₂ · 6 H₂O | 0.02 g/L |

Solution E: (1000 × Vitamin)

| | |
|---|---|
| Biotin | 0.20 g/L |

Solution F: (50 % w/v glucose)

| | |
|---|---|
| α-D(+)-Glucose · H₂O | 550.00 g/L |

Solution G: (Protocatechuic acid)

| | |
|---|---|
| Protocatechuic acid | 30.00 g/L |

To prepare the pre-culture minimal medium MM3, solutions A, B, C, and F described above were prepared separately and also separately sterilized at 120 °C for 20 min. Solutions D, E and G were separately prepared and sterile filtrated at 0.2 µm pore size. For 1 L of ready to use pre-culture medium MM3, 915 mL salts, 1 mL 1000 × Ca²⁺ stock solution, 1 mL 1000 × Mg²⁺ stock solution, 1 mL 1000 × TES, 1 mL 1000 × Vitamin stock solution, 1 mL of protocatechuic acid stock solution and 80 mL 50 % w/v glucose were mixed.

### Media and solutions - Preparation of minimal medium MM4 (CGXII-medium P-lim)

Solution A: Salts

| | |
|---|---|
| (NH₄)₂SO₄ | 10.93 g/L |
| Urea | 5.46 g/L |
| MOPS | 22.95 g/L |
| K₂HPO₄ | 0.011 g/L |
| KH₂PO₄ | 0.011 g/L |

pH was adjusted to pH 6.5 with 5M KOH
Solution B: (1000 × Ca²⁺)

| | |
|---|---|
| CaCl₂ · H₂O | 10.00 g/L |

Solution C: (1000 × Mg²⁺)

| | |
|---|---|
| MgSO₄ · 7 H₂O | 250.00 g/L |

Solution D: (1000 × Trace Elements Solution = TES)

| | |
|---|---|
| Fe(II)SO₄ · 7 H₂O | 16.40 g/L |
| MnSO₄ · H₂O | 10.00 g/L |
| CuSO₄ · 5 H₂O | 0.20 g/L |
| ZnSO₄ · 7 H₂O | 1.00 g/L |
| NiCl₂ · 6 H₂O | 0.02 g/L |

Solution E: (1000 × Vitamin)

| | |
|---|---|
| Biotin | 0.20 g/L |

Solution F: (50 % w/v glucose)

| | |
|---|---|
| α-D(+)-Glucose · H₂O | 550.00 g/L |

Solution G: (Protocatechuic acid)

| | |
|---|---|
| Protocatechuic acid | 30.00 g/L |

To prepare the pre-culture minimal medium MM4, solutions A, B, C, and F described above were prepared separately and also separately sterilized at 120 °C for 20 min. Solutions D, E and G were separately prepared and sterile filtrated at 0.2 µm pore size. For 1 L of ready to use pre-culture medium MM4, 915 mL salts, 1 mL 1000 × Ca²⁺ stock solution, 1 mL 1000 × Mg²⁺ stock solution, 1 mL 1000 × TES, 1 mL 1000 × Vitamin stock solution, 1mL of protocatechuic acid stock solution and 80 mL 50 % w/v glucose were mixed.

### Media and solutions - Preparation of minimal medium MM5 (CGXII-medium C-lim)

Solution A: Salts

| | |
|---|---|
| (NH₄)₂SO₄ | 3.28 g/L |
| Urea | 1.64 g/L |
| MOPS | 6.88 g/L |
| K₂HPO₄ | 0.33 g/L |
| KH₂PO₄ | 0.33 g/L |

pH was adjusted to pH 6.5 with 5M KOH
Solution B: (1000 × Ca²⁺)

| | |
|---|---|
| CaCl₂ · H₂O | 10.00 g/L |

Solution C: (1000 × Mg²⁺)

| | |
|---|---|
| MgSO₄ · 7 H₂O | 250.00 g/L |

Solution D: (1000 × Trace Elements Solution = TES)

| | |
|---|---|
| Fe(II)SO₄ · 7 H₂O | 16.40 g/L |
| MnSO₄ · H₂O | 10.00 g/L |
| CuSO₄ · 5 H₂O | 0.20 g/L |
| ZnSO₄ · 7 H₂O | 1.00 g/L |
| NiCl₂ · 6 H₂O | 0.02 g/L |

Solution E: (1000 × Vitamin)

| | |
|---|---|
| Biotin | 0.20 g/L |

Solution F: (50 % w/v glucose)

| | |
|---|---|
| α-D(+)-Glucose · H₂O | 550.00 g/L |

Solution G: (Protocatechuic acid)

| | |
|---|---|
| Protocatechuic acid | 30.00 g/L |

To prepare the pre-culture minimal medium MM5, solutions A, B, C, and F described above were prepared separately and also separately sterilized at 120 °C for 20 min. Solutions D, E and G were separately prepared and sterile filtrated at 0.2 µm pore size. For 300 mL of ready to use pre-culture medium MM5, 274.5 mL salts, 0.3 mL 1000 × Ca²⁺ stock solution, 0.3 mL 1000 × Mg²⁺ stock solution, 0.3 mL 1000 × TES, 0.3 mL 1000 × Vitamin stock solution, 0.3 mL of protocatechuic acid stock solution and 24 mL 50 % w/v glucose were mixed.

### Media and solutions - Preparation of minimal medium MM6 (CGXII-medium Transition)

Solution A: Salts

| | |
|---|---|
| (NH₄)₂SO₄ | 19.72 g/L |
| K₂HPO₄ | 1.97 g/L |
| KH₂PO₄ | 1.97 g/L |

Solution B: (1000 × Ca²⁺)

| | |
|---|---|
| CaCl₂ · H₂O | 10.00 g/L |

Solution C: (1000 × Mg²⁺)

| | |
|---|---|
| MgSO₄ · 7 H₂O | 250.00 g/L |

Solution D: (1000 × Trace Elements Solution = TES)

| | |
|---|---|
| Fe(II)SO₄ · 7 H₂O | 16.40 g/L |
| MnSO₄ · H₂O | 10.00 g/L |
| CuSO₄ · 5 H₂O | 0.20 g/L |
| ZnSO₄ · 7 H₂O | 1.00 g/L |
| NiCl₂ · 6 H₂O | 0.02 g/L |

Solution E: (1000 × Vitamin)

| | |
|---|---|
| Biotin | 0.20 g/L |

Solution F: (50 % w/v glucose)

| | |
|---|---|
| α-D(+)-Glucose · H₂O | 550.00 g/L |

Solution G: (Protocatechuic acid)

| | |
|---|---|
| Protocatechuic acid | 30.00 g/L |

To prepare the minimal medium MM6, solutions A, B, C, and F described above were prepared separately and also separately sterilized at 120 °C for 20 min. Solutions D,E and G were separately prepared and sterile filtrated at 0.2 µm pore size. For 1.7 L of ready to use minimal medium MM6, 1553 mL salts, 1.75 mL 1000 × Ca²⁺ stock solution, 1.75 mL 1000 × Mg²⁺ stock solution, 1.75 mL 1000 × TES, 1.75 mL 1000 × Vitamin stock solution, 1.75 mL of protocatechuic acid stock solution and 140 mL 50 % w/v glucose were mixed.

### Cultivation of C. glutamicum wild-type and FastA.

### Pre-culture cultivation I on agar plates

*C. glutamicum* cells from a glycerol cell culture stock where spread on the agar plate and the culture was incubated at 30 °C for 48 h.

### Pre-culture cultivation II in glass reaction tubes

Colonies extracted from agar plates of pre-culture cultivation I were used to inoculate the 2 × TY medium in glass reaction tubes and incubated for 8 h at 30 °C under constant agitation (120 min⁻¹).

### Pre-culture cultivation III in shaking flasks

Sterile 500 mL Erlenmeyer shaking flasks with baffles were filled with 50 mL of the minimal medium MM1. Three individual pre-culture minimal medium flasks each were inoculated with 5 mL broth of pre-culture cultivation II and incubated overnight 30 °C and constant agitation (120 min⁻¹).

### Pre-culture cultivation IV in shaking flasks (N-lim)

Sterile 3000 mL Erlenmeyer shaking flasks with baffles were filled with 333 mL of minimal medium MM3. Three individual pre-culture minimal medium flasks each were inoculated with 5 mL broth of pre-culture cultivation II and incubated for 84 h at 30 °C and constant agitation (120 min⁻¹).

### Pre-culture cultivation V in shaking flasks (P-lim)

Sterile 3000 mL Erlenmeyer shaking flasks with baffles were filled with 333 mL of minimal medium M4. Three individual pre-culture minimal medium flasks each were inoculated with 5 mL broth of pre-culture cultivation II and incubated for 48h at 30 °C and constant agitation (120 min⁻¹).

### Pre-culture cultivation VI in shaking flasks (C-lim)

Sterile 500 mL Erlenmeyer shaking flasks with baffles were filled with 50 mL of the minimal medium MM5. Six individual pre-culture minimal medium flasks each were inoculated with 5 mL broth of pre-culture cultivation II and incubated 84 h at 30 °C and constant agitation (120 min⁻¹).

### Main cultivation I in bioreactors (growth rate screening)

Main cultivations I were carried out in a parallel cultivation system consisting of three identical HWS glass bioreactors with a working volume of 250 mL each. After assemblage of the cultivation system, every bioreactor was separately filled with 179.6 mL Solution A of the minimal medium MM2, 0.2 mL Solution B and 0.2 mL Solution C. This salt solution was sterilized in every bioreactor at 120 °C for 20 min. After sterilization a total volume of 8 mL 50% w/v glucose stock solution, 0.2 mL 1000 × TES, and 0.2 mL 1000 × Vitamin stock solution was added sterile to every vessel. Each bioreactor was inoculated with 12 mL of a concentrated pre-culture giving a starting Optical Density of 1. Fermentations were performed at a constant temperature of 30 °C, agitation and good oxygen supply. pH was kept at a constant level of 7.4 by addition of 25 % [V/V] NH₄OH. The process length varied for the wild-type strain and the strain FastA. Individual fermentation durations are mentioned for the corresponding strains in Examples 1 and 2 below.

### Main cultivation II in bioreactors (N-lim)

Main cultivations II were carried out in a 3 L fermenter with a working volume of 1.75 liters. The reactor was equipped with one Rushton turbine, a pH, a pressure, and a dissolved oxygen sensor and a cooling and a heating rod. The fully assembled reactor was filled with sterilization buffer and autoclaved at 121 °C for 20 min. The sterilization buffer was then drained from the reactor in a sterile manner and the reactor was filled with 1.70 L of sterile minimal medium MM6. The reactor was then inoculated with 50 mL of concentrated pre-culture IV so that the resulting optical density OD₆₀₀ was 5.

Fermentations were performed at a constant temperature of 30 °C and a positive pressure of 50 kPa. Agitation was controlled so that the dissolved oxygen never fell below 30 % of the maximum possible value and pH was kept at a constant level of 7.4 by addition of 25 % [V/V] NH₄OH.

### Main cultivation III in bioreactors (P-lim)

Main cultivations III were carried out in a 3 L fermenter with a working volume of 1.75 liters. The reactor was equipped with one Rushton turbine, a pH, a pressure, and a dissolved oxygen sensor and a cooling and a heating rod. The fully assembled reactor was filled with sterilization buffer and autoclaved at 121 °C for 20 min. The sterilization buffer was then drained from the reactor in a sterile manner and the reactor was filled with 1.70 L of sterile minimal medium MM6.

The reactor was then inoculated with 50 mL of concentrated pre-culture V so that the resulting optical density OD₆₀₀ was 5.

Fermentations were performed at a constant temperature of 30 °C and a positive pressure of 50 kPa. Agitation was controlled so that the dissolved oxygen never fell below 30 % of the maximum possible value and pH was kept at a constant level of 7.4 by addition of 25 % NH₄OH [V/V].

### Main cultivation IV in bioreactors (C-lim)

Main cultivations IV were carried out in a 3 L fermenter with a working volume of 1.75 liters. The reactor was equipped with one Rushton turbine, a pH, a pressure, and a dissolved oxygen sensor and a cooling and a heating rod. The fully assembled reactor was filled with sterilization buffer and autoclaved at 121 °C for 20 min. The sterilization buffer was then drained from the reactor in a sterile manner and the reactor was filled with 1.70 L of sterile minimal medium MM6. The reactor was then inoculated with 50 mL of concentrated pre-culture VI so that the resulting optical density OD₆₀₀ was 5.

Fermentations were performed at a constant temperature of 30 °C and a positive pressure of 50 kPa. Agitation was controlled so that the dissolved oxygen never fell below 30 % of the maximum possible value and pH was kept at a constant level of 7.4 by addition of 25 % [V/V] NH₄OH.

### Example 1: Batch cultivations to screen growth rates of C. glutamicum wild-type

Every batch cultivation process with the aim to screen the strains started with identical conditions. The sole C-source glucose was always in vast excess at the beginning of every fermentation. This also extended to all additional nutrients in the batch minimal medium, as listed above. For the first couple of hours every C. *glutamicum* strain was growing exponentially at its very specific maximum growth rate and consumed glucose with its individual biomass-specific uptake rate under non-limited conditions. This state is termed as "Exponential Growth" in Figures 1 and 2. The fixed glucose concentration in the minimal medium enables the bacterial cells to form a certain total biomass before entering carbon-depleted nutritional conditions. The subsequent phase is further termed as "Stationary Phase" in Figures 1 and 2. During this late state of the fermentation process bacterial growth stopped due to carbon-depletion and no further biomass formation occurred. However, all additional nutrients remained abundantly.

In this example, *Corynebacterium glutamicum* ATCC13032 wild-type was cultivated under pre-culture conditions III in shaking flasks, as described above, for 12 hours with constant agitation. These bacterial cells were then transferred into the three bioreactors under sterile conditions to start the batch cultivation process. *C. glutamicum* wild-type cells were cultivated for a total period of 11 hours with the starting concentration of glucose being 20 g/L. In the exponential growth phase the maximal biomass-specific growth rate was 0.324 ± 0.003 h⁻¹ and glucose was consumed at a biomass-specific rate of 0.66 ± 0.04 g_{Glc}/g_{cdw}h (cdw: cell dry weight). As can be seen in Figure 1, these cells were growing exponentially during the first 10.5 hours of cultivation before all the carbon in the minimal medium was depleted and the stationary phase was reached. Subsequently, no further biomass formation occurred in the next half hour (process time 11 h) as can be seen in Figure 1.

### Example 2: Batch cultivations to screen growth rates of C. glutamicum mutant FastA

In this example, *Corynebacterium glutamicum* FastA was cultivated under pre-culture conditions III in shaking flasks, as described above, for 12 hours with constant agitation. These bacterial cells were then transferred into the three bioreactors under sterile conditions to start the batch cultivation process. C. *glutamicum* FastA cells were cultivated for a total period of 9.5 hours with the starting concentration of glucose being 20 g/L. In the exponential growth phase the maximal biomass-specific growth rate was 0.40 ± 0.02 h⁻¹ and glucose was consumed at a biomass-specific rate of 0.90 ± 0.06 g_{Glc}/g_{cdw}h (cdw: cell dry weight). As can be seen in Figure 2, these cells were growing exponentially during the first 8.5 hours of cultivation before all the carbon in the minimal medium was depleted and the stationary phase was reached. Subsequently, no further biomass formation occurred in the next one hour (process time 9.5 h) as can be seen in Figure 2.

Table 2 shows the comparison of biomass-specific rates in the *Corynebacterium glutamicum* wild-type and the FastA strain during the batch cultivation phase of exponential growth with all nutrients in excess. Values are calculated from three parallel fermentations n = 3. The respective data are illustrated in Figure 3.

**Table 2:**

| Strain | Glucose uptake qS, g/g_{cdw}h | | Growth µ, h⁻¹ | |
|---|---|---|---|---|
| | Ø | σ | Ø | σ |
| *C*. *glutamicum* ATCC13032 wild-type | 0.66 | 0.04 | 0.324 | 0.003 |
| *C. glutamicum* FastA | 0.90 | 0.06 | 0.40 | 0.02 |

### Example 3: Identification of regulators governing the growth rate transition in C. glutamicum A TCC13032

The growth rate transitions took place during main cultures II, III, IV. The same minimal medium MM6 and the same conditions were used in these experiments. The growth rate transitions were induced by the conditions during the previous pre-culture. Three different limiting conditions were used during the pre-cultures: Nitrogen limitation in pre-culture IV, phosphate limitation in pre-culture V and carbon limitation in pre-culture IV. The transitions were sampled, the differential growth rate was determined via cell dry weight measurement and the transcriptome was measured at the growth rates of 0, 0.1, 0.2, 0.3 and 0.4 h⁻¹ in case of main culture II (N-lim) and III (P-lim) and 0.1, 0.2, 0.3 and 0.4 h⁻¹ in case of main culture IV (C-lim). The log-fold changes of each gene, condition and growth rate were determined in reference to the sample with the highest growth rate. The resulting profiles of the gene expression levels were used to identify regulators which show a change in expression profile over the growth rate independent of the condition of the previous pre-culture.

In this example the identification of the regulators with influence on the growth rate in *Corynebacterium glutamicum* ATCC13032 is shown. Examples of gene expression profiles of regulators over the growth rate are shown in Figure 4. The profiles of several regulators show a similar response to the increase of the growth rate during the transition independent of the pre-culture condition. The indicated log-fold values are calculated from three biological replicates per condition.

### Discussion:

According to the present invention, an increased growth rate and increased sugar uptake rate have been achieved in exponentially growing cells by specific targeted interventions into *C. glutamicum* metabolism and metabolic regulation.

Concerning metabolic regulation, it is known that the master regulator RamA plays a central role in the coordinated control of central carbon metabolism of C. *glutamicum:* it is involved in acetate metabolism, in ethanol utilization, and it regulates the TCA cycle. Furthermore, RamA negatively controls expression of its own gene and activates expression of *ramB* (encoding the master regulator RamB of acetate metabolism) and of *sugR* encoding SugR, a sugar-responsive global regulator.

Several regulators which show a change in expression with an increase in the growth rate could be identified. One of these being RamA. These regulators could be identified as prime targets for strain improvement towards higher growth rates.

## Claims

1. A recombinant *Corynebacterium* sp. host cell, wherein said cell comprises at least one mutation in relation to a wild-type cell, wherein said mutation is selected from the group consisting of mutations that
(i) reduce the activity of a protein, selected from the group consisting of RamA, SigH, RshA, SigM, SufR, OxyR, SigE, SigA, and SigD; or
(ii) increase the activity of a protein, selected from the group consisting of RipA and DtxR.

2. The recombinant host cell according to claim 1, wherein said mutation is selected from the group consisting of mutations that
(i) reduce the activity of a protein, selected from the group consisting of RamA, SigH, RshA, SigM, SufR, OxyR, and SigE; or
(ii) increase the activity of a protein, selected from the group consisting of RipA and DtxR.

3. The recombinant host cell according to claim 1 or claim 2, wherein said mutation is selected from the group consisting of mutations that
(i) reduce the activity of the transcriptional regulator RamA (Regulator of acetate metabolism A); or
(ii) increase the activity of the transcriptional regulator DtxR (Diphteria toxin repressor).

4. The recombinant host cell according to claim 3, wherein said mutation is
(i) the amino acid substitution S101C in RamA (RamA[S101C]); or
(ii) the amino acid substitution R103H in DtxR (DtxR[R103H]).

5. The recombinant host cell according to any one of claims 1 to 4, wherein said host cell is a *Corynebacterium glutamicum* (*C. glutamicum)* host cell.

6. The host cell according to claim 5, wherein said host cell is derived from *C. glutamicum* strain *C. glutamicum* ATCC 13032.

7. A method for the biosynthetic production of a product of interest (POI), wherein said POI is produced in a recombinant host cell according to any one of claims 1 to 6.

8. The method according to claim 7, wherein said POI is a protein and said protein is expressed in said recombinant host cell.

9. The method according to claim 7, wherein said POI is an organic molecule and said organic molecule is produced in said recombinant host cell as a metabolite.

10. The method according to claim 9, wherein said organic molecule is an amino acid.

11. The method according to claim 10, wherein said amino acid is an L-amino acid.

12. The method according to claim 10 or claim 11, wherein said amino acid is selected from the group consisting of L-glutamic acid, L-lysine, L-ornithine, L-proline, L-arginine, L-citrulline, and gamma-butyric acid (GABA).

13. The method according to any one of claims 10 to 12, wherein the starting material for the production of said metabolite is a sugar.

14. The method of claim 13, wherein said sugar is selected from the group consisting of glucose, fructose, mannose, xylose, arabinose, and sucrose.

15. Use of a recombinant host cell according to any one of claims 1 to 6 for the biosynthetic production of a product of interest (POI).


SEQUENCE LISTING

<110>  Universität Stuttgart

<120>  Corynebacterium spp. strains having increased rates of growth
       and/or sugar uptake

<130>  S13725EU

<160>  8

<170>  PatentIn version 3.5

<210>  1
<211>  51
<212>  DNA
<213>  Artificial sequence

<220>
<223>  Amplification primer

<400>  1
aacagctatg accatgatta cgccaagctt gggggttaac tacctcttcg g                51

<210>  2
<211>  20
<212>  DNA
<213>  Artificial sequence

<220>
<223>  Amplification primer

<400>  2
cgacaatgca atgaaggccc                                                   20

<210>  3
<211>  20
<212>  DNA
<213>  Artificial sequence

<220>
<223>  Amplification primer

<400>  3
gggccttcat tgcattgtcg                                                   20

<210>  4
<211>  49
<212>  DNA
<213>  Artificial sequence

<220>
<223>  Amplification primer

<400>  4
agtgaattcg agctcggtac ccggggatcc ttaaggcagt gcgccgatc                   49

<210>  5
<211>  19

<212>  DNA
<213>  Artificial sequence

<220>
<223>  Sequencing primer

<400>  5
caggctttac actttatgc                                                    19


<210>  6
<211>  17
<212>  DNA
<213>  Artificial sequence

<220>
<223>  Sequencing primer

<400>  6
cccatctctt cagcagc                                                      17


<210>  7
<211>  17
<212>  DNA
<213>  Artificial sequence

<220>
<223>  Sequencing primer

<400>  7
gttgcaggac aatcgcc                                                      17


<210>  8
<211>  17
<212>  DNA
<213>  Artificial sequence

<220>
<223>  Sequencing primer

<400>  8
cccatctctt cagcagc                                                      17